# FASCICULE DE BREVET EUROPEEN

(11) **EP 3 322 836 B1**
(45) Date de publication et mention de la délivrance du brevet: **16.02.2022**
(21) Numéro de dépôt: 16747821.3
(22) Date de dépôt: 29.06.2016
(51) Int. Cl.: C23C 14/56, B65G 53/46, B65G 65/48, B65G 17/06, B65G 29/02

(54) **INSTALLATION DE TRAITEMENT COMPRENANT UN DISPOSITIF DE TRANSFERT ENTRE UNE ZONE A PRESSION ATMOSPHÉRIQUE ET UNE ZONE SOUS VIDE, ET PROCÉDÉ DE MISE EN OEUVRE CORRESPONDANT**
HANDHABUNGSEINRICHTUNG MIT EINER TRANSFERVORRICHTUNG, DIE ZWISCHEN EINER ZONE BEI ATMOSPHÄRISCHEM DRUCK UND EINER ZONE UNTER VAKUUM ÜBERTRÄGT, UND ZUGEHÖRIGES VERFAHREN ZUR IMPLEMENTIERUNG
HANDLING FACILITY COMPRISING A TRANSFER DEVICE TRANSFERRING BETWEEN A ZONE AT ATMOPSHERIC PRESSURE AND A ZONE UNDER VACUUM, AND CORRESPONDING METHOD OF IMPLEMENTATION

(30) Priorité: 13.07.2015 FR 1556641
(43) Date de publication de la demande: 23.05.2018
(73) Titulaire: Coating Plasma Innovation, 13710 Fuveau (FR)
(72) Inventeur: VIARD, Jocelyn, 13960 Sausset-les-Pins (FR)
(74) Mandataire: Schmidt, Martin Peter
(86) Numéro de dépôt international: PCT/FR2016/051612
(87) Numéro de publication internationale: WO 2017/009537

(56) Documents cités:
- CH-A- 437 219
- FR-A1- 2 740 761
- GB-A- 2 012 705
- US-A- 3 894 926

## Description

### Domaine technique de l'invention

L'invention concerne la technologie du vide et des traitements sous vide. Elle a trait à une installation de traitement sous vide équipée d'au moins un dispositif de transfert d'objets entre une zone à pression atmosphérique et une zone sous vide, ainsi qu'à un procédé de mise en oeuvre de cette installation de traitement. A titre d'exemple non limitatif, les objets peuvent être notamment des objets creux, possédant un volume intérieur délimité par une paroi présentant une face intérieure et une face extérieure. De tels objets sont entre autres des gobelets, des coupes, des barquettes, des pots, des tubes ou encore des moules industriels.

L'invention vise plus particulièrement une telle installation, dans laquelle les objets précités sont introduits dans l'enceinte de traitement sous vide par le dispositif de transfert, de manière continue et à cadence élevée.

### Art antérieur

Les procédés de traitement plasma sous vide nécessitent la mise sous vide des pièces à traiter. Dans l'art antérieur, la mise en place des objets est en général réalisée en « batch », ou en lots. Il s'agit d'un processus long, peu compatible avec des cadences élevées.

On citera tout d'abord, en tant que document représentatif de cet état de la technique, US 8 336 448. Après mise en place des objets dans la chambre plasma, celle-ci est mise sous vide afin de générer un type de plasma durant un temps de traitement déterminé.

On connait par ailleurs un procédé, dans lequel les objets à traiter stationnent dans des chambres plasma successives. Chaque chambre est fermée par des sas qui permettent de garder une pression sous vide dans chaque chambre, durant le traitement. Ce procédé présente des inconvénients car, durant le transfert d'une chambre à l'autre, les plasmas doivent être arrêtés. Le système des sas nécessite l'utilisation et la gestion de manipulateurs sous vide, ce qui complique l'appareillage. Par ailleurs, le traitement des objets en continu n'est pas réalisable, du moins de façon simple. On citera notamment, en tant que documents représentatifs de cet état de la technique, EP 2 630 271 ou encore DE 10 2008 019427.

On connait par ailleurs, de US 6 296 735, une installation de traitement par plasma. Cette dernière comprend une enceinte susceptible d'être mise sous vide, laquelle renferme des chambres plasma. De plus, un transporteur permet de déplacer les objets entre une zone de stockage amont et une zone de stockage aval. Cette installation ne permet pas de pallier les lacunes, inhérentes aux solutions industrielles décrites ci-dessus.

De plus on connait, de US 2011/308456, une installation de traitement équipée d'un barillet où sont disposées les pièces. Le support du barillet comprend en son centre un ensemble permettant la mise en oeuvre de traitements de surface. Lors de chaque traitement, le vide est fait dans l'enceinte. Ce document n'apporte pas une solution satisfaisante, car il ne permet pas l'alimentation en continu d'un nombre élevé de pièces à traiter.

On connait également un procédé de traitement plasma et de stérilisation, mettant en oeuvre un convoyeur de bouteilles en matière plastique. Ce convoyeur passe de manière successive à travers une chambre de mise sous vide, puis dans la zone de traitement et enfin dans une deuxième chambre de mise sous vide. Ce procédé permet de traiter en continu des bouteilles positionnées en file sur le convoyeur. Ce document n'apporte cependant pas une solution satisfaisante, car il implique une alimentation des pièces une par une. On citera notamment, en tant que document représentatif de cet état de la technique, US 2008/2630271.

Enfin on connaît, de US 3 894 926, un dispositif de transfert d'objets entre une zone à pression atmosphérique et une zone destinée à être mise sous vide. Ce dispositif comprend un bâti, un logement ménagé dans ce bâti, ainsi qu'un organe de transfert, mobile par rapport au bâti, qui comporte des cellules de réception des objets à transférer. 3 894 926 A divulgue ainsi une installation de traitement de surface selon le préambule de la revendication 1. Le bâti définit deux ouvertures en communication respectivement avec la zone à pression atmosphérique et la zone destinée à être mise sous vide, ainsi qu'une région intermédiaire destinée à être placée soit sous vide, soit à pression atmosphérique. Ce dispositif de transfert ne peut toutefois pas être intégré dans une installation de traitement telle que visée par l'invention, sans impliquer des difficultés techniques significatives.

Un objectif de la présente invention est de remédier, au moins partiellement, aux inconvénients de l'art antérieur évoqués ci-dessus.

Un autre objectif de l'invention est de proposer une installation de traitement sous vide équipée d'un dispositif industriel permettant de transférer, vers une zone de traitement sous vide, une grande quantité de pièces en un temps réduit.

Un autre objectif de l'invention est de proposer une telle installation dont le dispositif de transfert autorise une alimentation de type continu de ces pièces vers cette zone de traitement sous vide.

Un autre objectif de l'invention est de proposer une telle installation dont le dispositif de transfert présente une structure relativement simple en vue de réaliser l'alimentation et/ou la sortie des pièces vers/depuis la zone sous vide.

### Objet de l'invention

Selon l'invention, les objectifs ci-dessus sont atteints au moyen d'une installation de traitement de surface, comprenant :
- une enceinte principale sous vide équipée de moyens de traitement, notamment de type plasma,
- au moins un dispositif de transfert d'objets entre une zone à pression atmosphérique et une zone sous vide, le ou chaque dispositif de transfert étant prévu en amont et/ou aval de l'enceinte principale et comprenant :
   - un bâti
   - un logement ménagé dans ce bâti,
   - un organe de transfert, monté dans le logement de manière mobile par rapport au bâti en étant monté à rotation dans le logement, cet organe de transfert comprenant au moins une cellule de réception d'au moins un objet à transférer,
   - le bâti définissant une première ouverture en communication avec la zone à pression atmosphérique, une deuxième ouverture en communication avec la zone sous vide, ainsi qu'une région intermédiaire placée soit sous vide, soit à pression atmosphérique,
   - des moyens d'isolation entre la région intermédiaire et chaque ouverture,
   - des moyens d'entrainement propres à entrainer chaque cellule successivement en regard de la première ouverture, de la région intermédiaire, puis de la deuxième ouverture,
l'installation comprenant en outre :
- des moyens de dépilage/empilage d'objets, placés en regard de la deuxième ouverture de chaque dispositif de transfert, et où l'enceinte principale de l'installation comprend:
- des moyens de convoyage des objets,
- des éléments de support de chaque objet, solidaires des moyens de convoyage.

Selon d'autres caractéristiques de l'installation de traitement de l'invention, prises isolément ou selon toute combinaison techniquement compatible :
- les moyens d'isolation sont formés par une restriction de section entre des parois en regard appartenant respectivement au bâti et à l'organe de transfert.
- la distance entre les parois en regard, appartenant respectivement au bâti et à l'organe de transfert, est inférieure à 200 micromètres, en particulier inférieure à 100 micromètres, plus particulièrement voisine de 50 micromètres.
- au moins un canal débouchant sur la région intermédiaire et associé à des moyens de pompage, pour la mise sous vide de la cellule en regard de ladite région intermédiaire.
- en vue transversale, l'organe de transfert comprend plusieurs cellules, en particulier quatre cellules, réparties angulairement de façon régulière.
- en vue de face, l'organe de transfert comprend plusieurs cellules réparties longitudinalement de façon régulière.
- les moyens de dépilage/empilage comprennent deux organes de dépilage placés l'un au-dessus de l'autre, chaque organe étant mobile entre une position de retenue, dans laquelle il s'oppose à la chute des objets, et une position de libération, dans laquelle ils permettent la chute des objets par gravité.
- chaque organe de dépilage comprend un corps allongé creusé d'échancrures, notamment semi-circulaires, réparties de manière régulière.
- les moyens de convoyage comprennent au moins deux bandes de convoyage parallèles, et plusieurs éléments de support sont regroupés les uns à côté des autres au sein d'un organe de support, s'étendant transversalement entre au moins deux bandes voisines.
- chaque élément de support comprend des moyens de centrage de l'objet, répartis autour d'une ouverture centrale.

L'invention a en outre pour objet un procédé de mise en oeuvre de l'installation ci-dessus, dans lequel on place une première pile d'objets dans une première cellule en regard de la zone à pression atmosphérique, on déplace l'organe de transfert de sorte que la première cellule soit en regard de la région intermédiaire, on met sous vide la première cellule en regard de la région intermédiaire, on déplace l'organe de transfert de sorte que la première cellule soit en regard de la zone sous vide, on dépose la première pile d'objets sur le système de dépilage, on dépile les objets de la première pile, on dépose chaque objet ainsi dépilé sur un élément de support respectif.

Selon d'autres caractéristiques du procédé de mise en oeuvre de l'invention, prises isolément ou selon toute combinaison techniquement compatible :
- en même temps qu'on met sous vide la première cellule en regard de la région intermédiaire, on place une deuxième pile d'objets dans une deuxième cellule en regard de la zone à pression atmosphérique.
- en même temps qu'on met sous vide la deuxième cellule en regard de la région intermédiaire, on place une troisième pile d'objets dans une troisième cellule en regard de la zone à pression atmosphérique.
- on dépose chaque objet sur le système de dépilage par gravité.
- on met sous vide la cellule en regard de la région intermédiaire, pendant une durée comprise entre 10 et 60 secondes, de préférence entre 20 et 40 secondes.
- on met sous vide la cellule en regard de la région intermédiaire, à une pression inférieure à 5.10⁻² mbar, de préférence à une pression inférieure à 10⁻² mbar.

L'invention permet d'atteindre les objectifs précédemment mentionnés.

Le dispositif de transfert utilisé dans l'invention fait appel à une zone se trouvant en permanence à pression atmosphérique, ainsi qu'à une zone se trouvant en permanence sous vide. Dans ces conditions, seule une région intermédiaire doit faire l'objet d'une mise sous vide périodique. Par conséquent, cette opération de mise sous vide concerne un volume restreint, de sorte qu'elle peut être réalisée de façon commode et rapide.

Ce dispositif de transfert autorise donc une alimentation en continu de l'enceinte de traitement, placée en aval. Un nombre élevé d'objets peuvent être admis dans cette enceinte, via le dispositif conforme à l'invention. En outre les moyens de dépilage/empilage d'objets au sein de la chambre sous vide permettent le stockage temporaire d'objets, en attente d'être transportés par les moyens de convoyage dans l'enceinte principale.

De plus, ce dispositif de transfert présente une structure mécanique relativement simple. En particulier, on notera qu'il fait appel à un nombre réduit de pièces en mouvement. Dans ces conditions, les risques de dysfonctionnement sont réduits, ce qui évite les arrêts intempestifs du traitement sous vide.

### Description des figures

L'invention va être décrite ci-après, en référence aux dessins annexés, donnés uniquement à titre d'exemples non limitatifs, dans lesquels:
Les figures 1 et 2 sont des vues générales en perspective d'une installation conforme à l'invention, respectivement avec et sans carter de protection.
La figure 3 est une vue en perspective, analogue à la figure 2, illustrant plus en détails l'installation conforme à l'invention.
La figure 4a est une vue de face, illustrant l'installation conforme à l'invention.
La figure 4b est une vue de dessus, illustrant les barres d'un dépileur appartenant à l'installation conforme à l'invention.
Les figures 4c à 4f sont des vues de face et des vues de dessus, illustrant de façon schématique la mise en oeuvre du dépileur appartenant à l'installation conforme à l'invention.
La figure 5 est une vue en perspective, analogue à la figure 2, illustrant plus en détails les moyens de convoyage de l'installation conforme à l'invention.
La figure 6 est une vue en coupe transversale illustrant l'intérieur du dispositif de transfert appartenant à l'installation des figures précédentes.
Les figures 7a à 7e sont des vues en coupe transversale, illustrant de façon schématique la mise en oeuvre du dispositif de transfert de la figure 6.

Les références numériques suivantes sont utilisées dans la présente description :

| | |
|---|---|
| 1 enceinte principale | 50 chambre d'interface |
| 10 convoyeur | 11-14 bande sans fin |
| 20 support | 30 dépileur |
| 19 entretoise | 18 cylindre |
| 15 partie plane | 16 ergot |
| 21 languette | 23 pied |
| 22 plateau | 24 évidement circulaire |
| 25 picot | 31, 32 barre de dépilage |
| 34,34' corps allongé | 35,35' paroi |
| 51 bâti | 52,53 ouverture |
| 55 barillet | 54 canaux horizontaux |
| 56-59 cellule | C1-C12 cellule longitudinale |
| 60, 81, 82 moteur | 80 axe transversal |
| 90, 90' pointe | CO1,CO2 collerette |
| SR sens de rotation | SP sens de progression |
| ST sens de translation | ST1, ST2, ST3 translation |
| P1, P2, P3, P4 Pièce | F1, F2 rotation |

### Description détaillée

L'installation conforme à l'invention comprend essentiellement une enceinte principale 1 sous vide permettant le traitement de surface de pièces, ou objets, ainsi qu'une chambre d'interface 50. Cette dernière, qui forme un dispositif de transfert au sens de l'invention, permet l'alimentation et la mise sous vide de ces pièces.

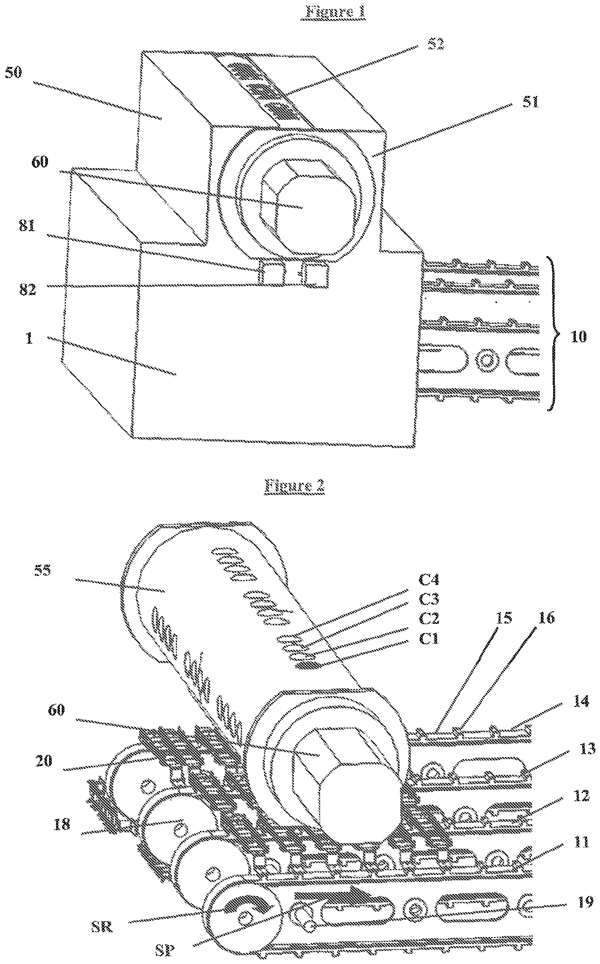

Tout d'abord, comme montré sur les figures 1 & 2, l'enceinte principale 1 comprend :
- des moyens de traitement de surface des pièces. Ces moyens, de type connu en soi, ne sont pas représentés. Il s'agit notamment de moyens de traitement de surface de type plasma ;
- un convoyeur 10 composé de quatre bandes (11, 12, 13, 14) sans fin équipées de moyens d'entrainement de type connu en soi et non représentés ;
- des organes de support 20, composé de plusieurs éléments de support dont la forme est adaptée à celle des pièces ;
- un dépileur 30 permettant de dépiler les pièces à traiter depuis la chambre 50 puis de les placer sur les supports 20.

Le convoyeur 10, figures 2, 3 & 5, comprend quatre bandes sans fin (11, 12, 13, 14) sur lesquelles sont fixés les supports de pièces 20. Ces bandes sont espacées les unes des autres d'une distance identique, au moyen d'entretoises 19. Les bandes sans fin (11, 12, 13, 14) sont équipées, à leurs extrémités, d'un cylindre 18 permettant leur actionnement. Le sens de rotation SR de ces cylindres définit le sens de progression SP de la bande sans fin.

La forme et les dimensions des bandes sans fin (11, 12, 13, 14) sont adaptées aux supports 20 et aux objets à traiter. La bande (11, 12, 13, 14) comprend une partie plane 15 et des ergots 16 disposés régulièrement le long de la partie plane 15. Ces ergots 16 permettent de solidariser les supports de pièces 20 sur les bandes.

Le convoyeur 10 s'étend sur l'ensemble de l'installation conforme à l'invention, depuis la chambre d'interface amont 50 en passant par la ou les zones de traitement plasma et jusqu'à la chambre d'interface aval. Cette dernière, qui n'est pas représentée, présente par exemple une structure analogue à la chambre amont 50. Le convoyeur est compris dans la chambre principale qui est elle-même sous vide.

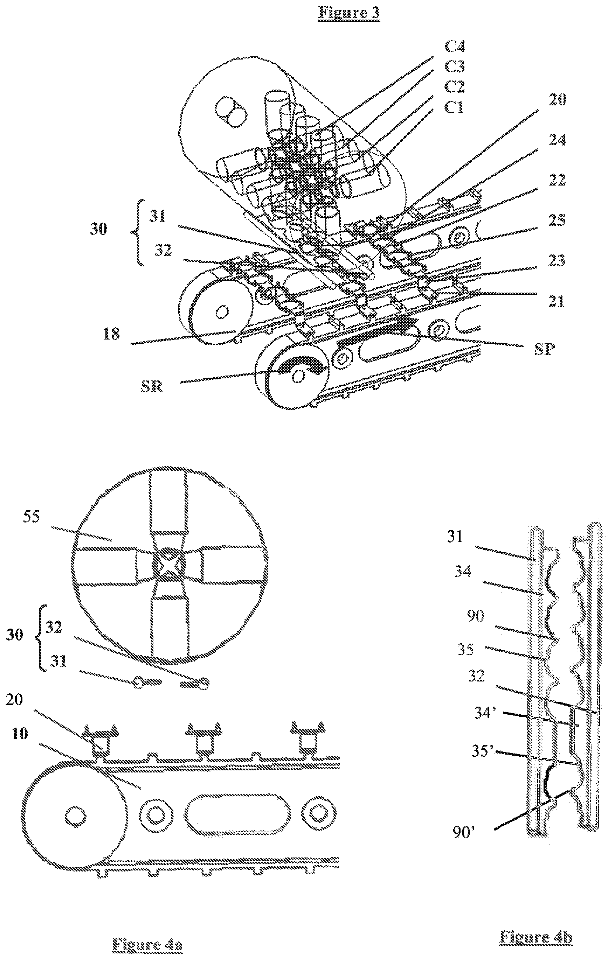

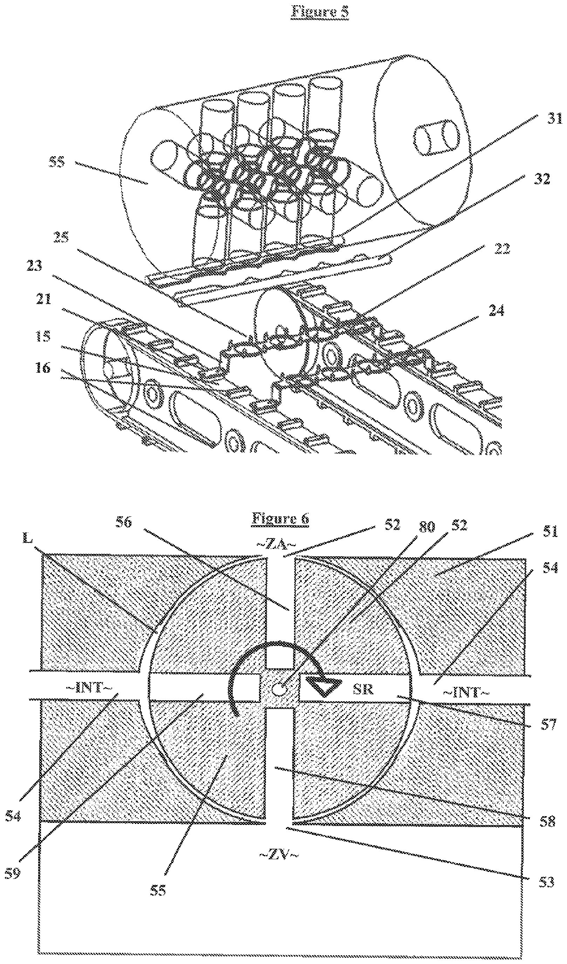

Dans l'exemple illustré figures 3 et 5, chaque organe de support 20 relie transversalement les bandes sans fin (11, 12, 13, 14). Cet organe de support, qui a la forme d'un banc, comprend plusieurs plateaux 22 de forme allongée, dont chacun est prolongé à ses deux extrémités par deux pieds 23. Chaque pied est en outre terminé par une languette 21 latérale permettant la fixation, par tout moyen approprié, sur un ergot d'une bande sans fin (11, 12, 13, 14).

Chaque plateau 22, figure 5, est creusé de quatre évidements circulaires 24 disposés de manière régulière sur sa longueur. Chaque évidement circulaire 24 est bordé, à sa périphérie, par quatre picots 25 diamétralement opposés. Ces picots forment des éléments de support, destinés à maintenir et centrer la pièce à traiter. Les évidements 24 prévus sur le plateau 22 permettent un traitement de surface optimal de la pièce, notamment en ce qu'ils délimitent des passages permettant au plasma d'atteindre, non seulement la face extérieure, mais aussi la face intérieure des pièces à traiter.

Le poste 30, figures 4a et 4b, est destiné au dépilage des objets à traiter. La forme et les dimensions de ce dépileur 30 sont adaptées aux objets à traiter. Dans l'exemple illustré, ce dépileur 30 comprend tout d'abord deux barres de dépilage 31 et 32, mobiles en translation selon un mouvement de va-et-vient. Les deux barres sont parallèles et leur écartement est adapté aux dimensions des pièces à traiter. Comme on le verra dans ce qui suit, l'action de dépilage est possible par le positionnement de la barre 31 au-dessus de la barre 32. Les deux barres 31 et 32 ont une structure identique et sont disposées de manière symétrique, de part et d'autre d'un axe parallèle à celui des supports 20. Les barres comprennent un corps allongé 34 creusé d'échancrures semi-circulaires, prévues de manière régulière sur la longueur.

Les parois 35, bordant ces échancrures, servent de maintien pour les pièces à traiter. Le poste 30 se trouve à une distance réduite du convoyeur, ce qui permet le dépilage en limitant le risque de casse des pièces. La distance entre le dépileur 30 et la chambre d'interface 50 peut lui conférer un rôle de réservoir intermédiaire, afin de ne pas compromettre la cadence élevée de traitement de l'enceinte principale. Les dispositifs de dépilage 31 et 32 comprennent chacun, à leur extrémité, un moteur d'entraînement respectif 81 et 82, comme visible sur la figure 1. Le sens de déplacement est défini par la flèche SP, sur la figure 2.

Ensuite, la chambre d'interface 50 permettant la mise sous vide comprend un bâti 51 de forme allongée, un barillet 55 reçu dans un logement L de ce bâti, ainsi qu'une pompe de mise sous vide, de type connu en soi, qui n'est pas représentée.

La forme et les dimensions du bâti 51 sont adaptées aux objets à traiter. Dans l'exemple illustré, figure 6, le bâti 51 de forme rectangulaire permet l'introduction des pièces sur la partie supérieure par l'intermédiaire d'une ouverture allongée 52, dite d'introduction. Cette dernière débouche sur l'extérieur, de sorte qu'elle est dans une zone à pression atmosphérique ZA. La partie inférieure du bâti 51 est creusée d'une ouverture dite d'évacuation 53, qui débouche sur l'enceinte principale qui forme une zone sous vide ZV.

Le bâti est en outre creusé de deux canaux 54 horizontaux, qui délimitent deux zones intermédiaires INT, disposés entre les ouvertures d'introduction et d'évacuation. Ces canaux sont connectés à une pompe de mise sous vide non représentée pouvant être intégré au bâti ou être extérieure à celui-ci. L'assemblage de la pompe avec les canaux 54 va définir la zone intermédiaire précitée susceptible d'être, soit à pression atmosphérique, soit sous vide. Cette fonction ressortira clairement du mode de fonctionnement qui sera explicité dans ce qui suit.

Le bâti comprend un axe transversal 80 supportant le barillet 55, qui forme un organe de transfert au sens de l'invention. Les parois en regard du barillet et du bâti définissent un jeu fonctionnel, qui est compris entre 20 et 200 micromètres, de préférence entre 50 et 100 micromètres, en particulier de l'ordre de 50 micromètres.

Cette restriction de section entre les parois en regard du barillet et du bâti forme des moyens d'isolation, permettant de limiter les échanges entre, d'une part, les régions intermédiaires et, d'autre part, la zone de pression atmosphérique ou la zone sous vide. En d'autres termes, la mise respectivement sous vide ou à pression atmosphérique des régions intermédiaires n'est sensiblement pas gênée par l'existence de la zone respectivement à pression atmosphérique ou sous vide. En alternative à cette restriction de section, ou en complément de celle-ci, les moyens d'isolation peuvent comprendre des joints placés sur les parois du bâti et/ou du barillet.

La forme et les dimensions du barillet 55 sont adaptées aux objets à traiter. Dans l'exemple illustré, le barillet 55 est un cylindre comprenant, en coupe transversale, quatre cellules 56,57,58,59 réparties angulairement de façon régulière, à la périphérie du barillet 55. Par ailleurs le barillet 55 comprend plusieurs cellules selon sa dimension longitudinale, en l'occurrence douze cellules référencées de C1 à C12. Le barillet 55 comprend un moteur d'entrainement 60, à son extrémité. Le sens de rotation est défini par la flèche SR.

Nous décrivons maintenant la mise en oeuvre de l'installation, décrite ci-dessus en référence aux figures 1 à 6.

On utilise des pièces présentant un voile tronconique dont la première extrémité est fermée par un fond et dont la deuxième extrémité ouverte est prolongée radialement par une collerette. De façon typique, cette pièce est donc par exemple un gobelet.

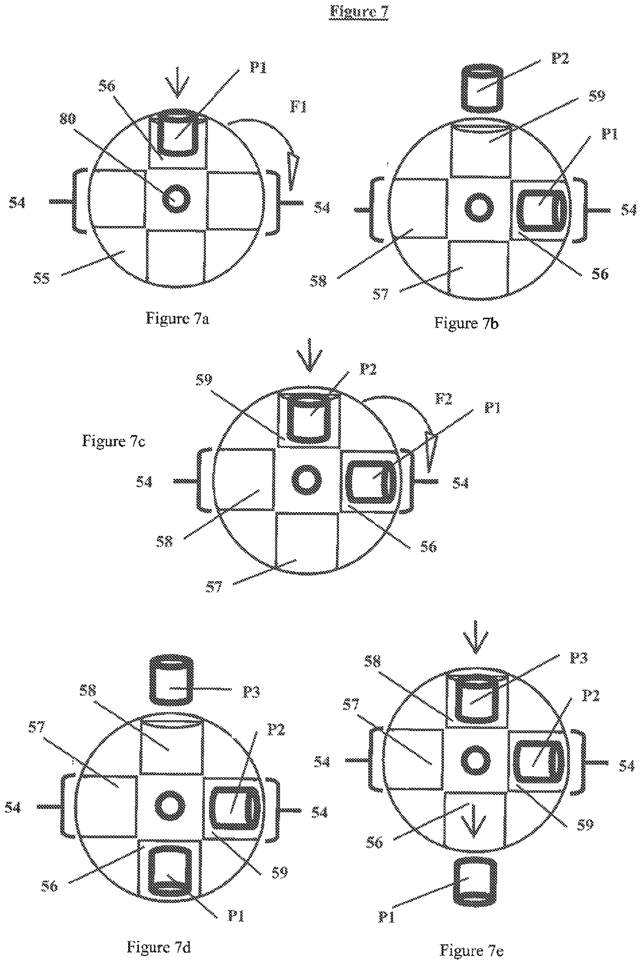

La mise en oeuvre du dispositif de transfert est décrite aux figures 7a à 7e, en référence à une cellule unique, dans la direction longitudinale. Toutefois, cette mise en oeuvre est simultanée pour tous les gobelets placés longitudinalement les uns derrière les autres, dans les cellules C1 à C12.

Le procédé comprend les étapes de mise en oeuvre suivantes :
A l'étape 1 figure 7a, la cellule 56 du barillet 55 reçoit un ensemble, ou pile, de pièces P1 à travers l'ouverture 52 du bâti 51. Cette pile, qui peut comprendre une à plusieurs dizaines de pièces, se trouve dans une zone à pression atmosphérique. Après une durée dont la valeur est expliquée dans ce qui suit, le barillet 55 est actionné en rotation selon l'axe central 80 d'un quart de tour, selon la flèche F1.
A l'étape 2, figure 7b, la cellule 56 arrive dans la région intermédiaire en face du canal 54. L'ensemble de pièces P1, qui est alors en regard de la région INT, passe d'un état de pression atmosphérique vers un état sous vide, figure 7c. Lors de cette mise sous vide, le volume intérieur de la cellule est placé à une pression inférieure à 5.10⁻² mbar, de préférence à une pression inférieure à 10⁻² mbar.
   On notera que, de façon avantageuse, la pompe de mise sous vide associée au canal 54 est activée de manière continue. Lorsqu'aucune cellule n'est placée en regard du canal, la pompe exerce une aspiration en regard d'une partie pleine, à savoir le corps du barillet, ce qui n'a aucun effet significatif. Dans le même temps, la cellule adjacente 59 est chargée avec une autre pile de pièces P2. Après une durée dont la valeur est expliquée dans ce qui suit, le barillet 55 est actionné en rotation d'un quart de tour, selon la flèche F2.
A l'étape 3, figure 7d, la cellule 56 se positionne à la verticale en face du dépileur 30, la cellule 59 se positionne en face du canal 54. Comme le montre la figure 7e, les pièces de la première pile P1 tombent par gravité vers le poste 30, selon la flèche G. De façon simultanée, la pile de pièces P2 passe d'un état de pression atmosphérique vers un état sous vide, comme décrit ci-dessus pour la pile P3, alors que la cellule suivante 58 est chargée d'une troisième pile de pièces P3. Après une durée dont la valeur est expliquée dans ce qui suit, le barillet 55 est actionné en rotation d'un quart de tour.

Puis la pile de pièces P2 tombe par gravité, depuis la cellule 59 vers le poste 30 de dépilage. De manière simultanée, les pièces P3 de la cellule 58 passent d'un état de pression atmosphérique vers un état sous vide, alors que la cellule 57 est chargée d'une pile supplémentaire de pièces, non représentée. La cellule 56 du barillet 55 revient ensuite à son point de départ, à savoir en position haute.

Le cycle des actions au niveau des différentes cellules 56 à 59 est donc identique, en étant décalé temporellement. On notera qu'il est avantageux de prévoir quatre cellules, car cela permet une introduction des pièces à pression atmosphérique, ainsi qu'une évacuation des pièces sous vide, réalisées toutes deux par gravité.

Les temps de transfert et d'action sur les cellules (56, 57, 58, 59) sont définis comme suit :
- le temps de rotation/transfert se situe typiquement dans une fourchette allant de deux à six secondes ;
- le temps de travail sur une cellule dépend du volume de la cellule. Plus ce volume est grand, plus le temps de pompage pour mettre sous vide celle-ci sera grand. Dans l'exemple décrit ci-dessus, le temps nécessaire est typiquement d'une trentaine de secondes afin de réaliser le passage de la pression atmosphérique au vide.

Le dépileur 30 permet l'alimentation des éléments de support entrainés par les quatre bandes sans fin (11, 12, 13, 14). Son mode de fonctionnement est le suivant.

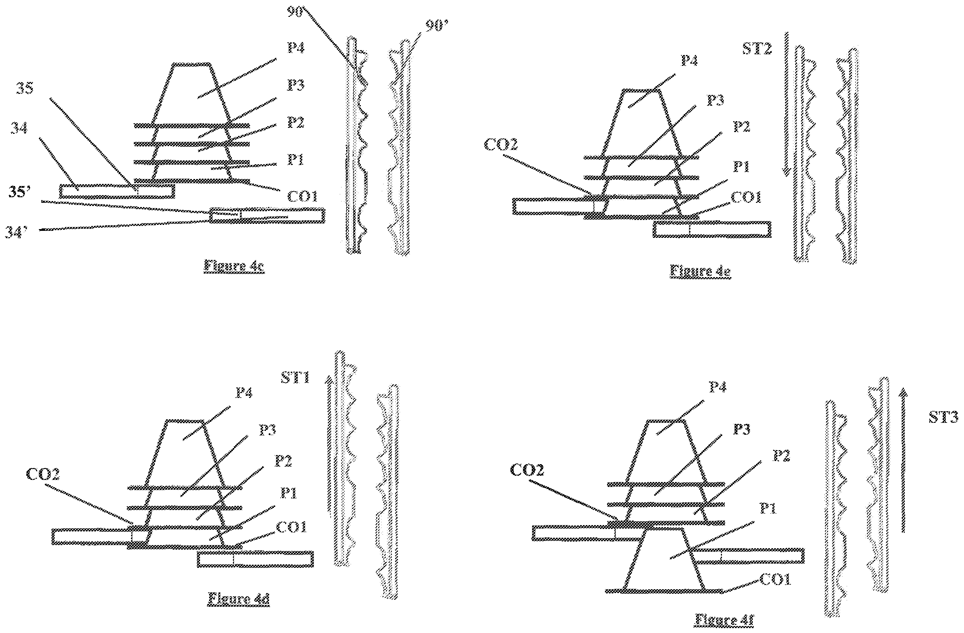

On suppose qu'une pile de pièces P1 à P4 vient de tomber par gravité depuis une des cellules de l'organe de transfert 50, voir figure 4c. La collerette CO1 de la pièce inférieure P1 se trouve alors en appui sur une pointe 90 d'une échancrure 35, ménagée dans la barre supérieure 31 de dépilage. Cette barre 31 est dans sa position initiale, dans laquelle elle retient les pièces, à savoir qu'elle s'oppose à leur chute par gravité.

Puis, comme le montre la figure 4d, on met en translation la barre 31 selon la flèche ST1, de façon à la déplacer depuis sa position initiale de retenue ci-dessus, vers une position dite escamotée ou de libération. La pile P1-P4 tombe alors par gravité, et la collerette CO1 de la première pièce P1 bute contre une pointe 90' d'une échancrure 35' de la barre inférieure de dépilage 32. Cette dernière se trouve dans sa position initiale de retenue, au sens défini immédiatement ci-dessus.

Ensuite, voir figure 4e, la barre de dépilage supérieure vient se repositionner dans sa position initiale de retenue, selon la flèche ST2, entre la collerette CO1 de la première pièce P1 et celle CO2 de la deuxième pièce P2. Puis, voir figure 4f, la barre de dépilage inférieure 32 est ensuite déplacée selon la flèche ST3 vers sa position escamotée de libération, ce qui permet à la pièce P1 de tomber par gravité vers un support 20. Puis la barre de dépilage inférieure 32 revient dans sa position initiale de retenue, opération qui n'est pas représentée dans les figures.

La séquence se répète indéfiniment. Le dépilage s'opère de manière synchronisée avec le sens de progression SR des quatre bandes sans fin (11, 12, 13,), afin de déposer les pièces sur les organes de support 20.

Comme évoqué plus haut, le dépileur sert de réservoir intermédiaire de pièces. Lorsque la cellule 56 est située au dessus du dépileur 30 et dépose les pièces, l'action de pompage s'opérant sur la cellule 57 suivante, permet de dépiler l'excédent de pièces limitant la rotation du barillet 55. Il reste alors quelques pièces sur le dépileur permettant la fourniture de pièces sur les supports, jusqu'à ce que la cellule 57 après rotation du barillet alimente à nouveau le dépileur.

Dans l'exemple ci-dessus, la chambre de transfert 50 se situe en amont dans l'installation. Toutefois on peut prévoir une chambre de transfert aval permettant l'extraction des pièces ainsi traitées, hors de l'enceinte principale 1. Dans ce cas, cette chambre est placée « tête en bas » par rapport à celle illustrée notamment en figure 6. Les pièces traitées tombent par exemple par gravité dans une cellule du barillet, placée dans la zone sous vide. Puis le barillet est mis en rotation, de sorte que ces pièces sont remises à pression atmosphérique dans la région intermédiaire. Enfin ces pièces sont évacuées de cette cellule, typiquement par gravité, vers la zone à pression atmosphérique.

## Revendications

1. Installation de traitement de surface, comprenant :
- une enceinte principale (1) sous vide équipée de moyens de traitement, notamment de type plasma,
- au moins un dispositif de transfert d'objets entre une zone à pression atmosphérique (ZA) et une zone sous vide (ZV), le ou chaque dispositif de transfert étant prévu en amont et/ou aval de l'enceinte principale et comprenant :
- un bâti (51)
- un logement (L) ménagé dans ce bâti,
- un organe de transfert (55), monté dans le logement de manière mobile par rapport au bâti en étant monté à rotation dans le logement (L), cet organe de transfert comprenant au moins une cellule (56-59) de réception d'au moins un objet à transférer,
- le bâti définissant une première ouverture (52) en communication avec la zone à pression atmosphérique, une deuxième ouverture (53) en communication avec la zone sous vide, ainsi qu'une région intermédiaire (INT) placée soit sous vide, soit à pression atmosphérique,
- des moyens d'isolation entre la région intermédiaire et chaque ouverture,
- des moyens d'entrainement (60), propres à entrainer chaque cellule successivement en regard de la première ouverture, de la région intermédiaire, puis de la deuxième ouverture,
**caractérisée en ce que** l'installation comprend en outre :
- des moyens (30) de dépilage/empilage d'objets, placés en regard de la deuxième ouverture de chaque dispositif de transfert,
et **en ce que** l'enceinte principale de l'installation comprend :
- des moyens (11-14) de convoyage des objets,
- des éléments (24, 25) de support de chaque objet, solidaires des moyens de convoyage.

2. Installation de traitement de surface selon l'une quelconque des revendications précédentes, **caractérisée en ce que** les moyens d'isolation sont formés par une restriction de section entre des parois en regard appartenant respectivement au bâti (51) et à l'organe de transfert (55).

3. Installation de traitement de surface selon l'une quelconque des revendications précédentes, **caractérisée en ce qu'**elle comprend au moins un canal (54) débouchant sur la région intermédiaire et associé à des moyens de pompage, pour la mise sous vide de la cellule en regard de ladite région intermédiaire.

4. Installation de traitement de surface selon l'une quelconque des revendications précédentes, **caractérisée en ce que**, en vue transversale, l'organe de transfert (55) comprend plusieurs cellules (56-59), en particulier quatre cellules, réparties angulairement de façon régulière.

5. Installation de traitement de surface selon l'une quelconque des revendications précédentes, **caractérisée en ce que**, en vue de face, l'organe de transfert (55) comprend plusieurs cellules (C1-C12), réparties longitudinalement de façon régulière.

6. Installation de traitement de surface selon l'une quelconque des revendications précédentes, **caractérisée en ce que** la distance entre les parois en regard, appartenant respectivement au bâti et à l'organe de transfert, est inférieure à 200 micromètres, en particulier inférieure à 100 micromètres, plus particulièrement voisine de 50 micromètres.

7. Installation de traitement de surface selon l'une quelconque des revendications précédentes, **caractérisée en ce que** les moyens de convoyage comprennent au moins deux bandes de convoyage parallèles (11-14), et plusieurs éléments de support (24, 25) sont regroupés les uns à côté des autres au sein d'un organe de support (20), s'étendant transversalement entre au moins deux bandes voisines.

8. Installation de traitement de surface selon l'une quelconque des revendications précédentes, **caractérisée** en ce les moyens de dépilage/empilage comprennent deux organes de dépilage (31, 32) placés l'un au-dessus de l'autre, chaque organe étant mobile entre une position de retenue, dans laquelle il s'oppose à la chute des objets, et une position de libération, dans laquelle ils permettent la chute des objets par gravité.

9. Installation de traitement de surface selon la revendication précédente, **caractérisée en ce que** chaque organe de dépilage comprend un corps allongé (34) creusé d'échancrures, notamment semi-circulaires, réparties de manière régulière.

10. Procédé de mise en œuvre de l'installation selon l'une quelconque des revendications précédentes, dans lequel :
- on place une première pile d'objets (P1) dans une première cellule (56) en regard de la zone à pression atmosphérique (ZA),
- on déplace l'organe de transfert de sorte que la première cellule soit en regard de la région intermédiaire (INT),
- on met sous vide la première cellule en regard de la région intermédiaire,
- on déplace l'organe de transfert de sorte que la première cellule soit en regard de la zone sous vide (ZV),
- on dépose la première pile d'objets sur le système de dépilage,
- on dépile les objets de la première pile,
- on dépose chaque objet ainsi dépilé sur un élément de support respectif.

11. Procédé selon la revendication précédente, **caractérisé en ce qu'**on met sous vide la cellule en regard de la région intermédiaire, à une pression inférieure à 5.10⁻² mbar, de préférence à une pression inférieure à 10⁻² mbar.

12. Procédé selon la revendication 10 ou 11, **caractérisé en ce que**, en même temps qu'on met sous vide la première cellule en regard de la région intermédiaire, on place une deuxième pile d'objets dans une deuxième cellule en regard de la zone à pression atmosphérique.

13. Procédé selon la revendication 12, **caractérisé en ce que**, en même temps qu'on met sous vide la deuxième cellule en regard de la région intermédiaire, on place une troisième pile d'objets dans une troisième cellule en regard de la zone à pression atmosphérique.

14. Procédé selon l'une des revendications 10 à 13, **caractérisé en ce qu'**on met sous vide la cellule en regard de la région intermédiaire, pendant une durée comprise entre 10 et 60 secondes, de préférence entre 20 et 40 secondes.

## Patentansprüche

1. Stationäre Handhabungseinrichtung, umfassend:
- eine Hauptkammer (1) unter Vakuum, die mit Handhabungsmitteln ausgestattet ist, besonders vom Typ Plasma,
- mindestens eine Transfervorrichtung für Objekte zwischen einer Zone bei atmosphärischem Druck (ZA) und einer Zone unter Vakuum (ZV), wobei die oder jede Transfervorrichtung der Hauptkammer vorgeschaltet und/oder nachgeschaltet vorgesehen ist und umfasst:
- einen Rahmen (51),
- ein Gehäuse (L), das im Rahmen vorgesehen ist,
- ein Transferelement (55), das im Gehäuse beweglich relativ zum Rahmen montiert ist, wobei es drehbar im Gehäuse (L) montiert ist, wobei dieses Transferelement mindestens eine Aufnahmezelle (56-59) von mindestens einem zu befördernden Objekt umfasst,
- wobei der Rahmen eine erste Öffnung (52) bildet in Verbindung mit der Zone bei atmosphärischem Druck, eine zweite Öffnung (53) in Verbindung mit der Zone unter Vakuum sowie einen Zwischenbereich (INT), der entweder unter Vakuum oder unter atmosphärischem Druck steht,
- Isoliermittel zwischen dem Zwischenbereich und jeder Öffnung,
- Antriebsmittel (60), geeignet, um jede Zelle anzutreiben, die nacheinander der ersten Öffnung, dem Zwischenbereich und dann der zweiten Öffnung zugewandt ist,
**dadurch gekennzeichnet, dass** die Einrichtung ferner umfasst:
- Mittel (30) zum Entstapeln/Stapeln von Objekten, die der zweiten Öffnung jeder Transfervorrichtung zugewandt angeordnet sind,
und dass die Hauptkammer der Einrichtung umfasst:
- Mittel (11-14) zum Befördern der Objekte,
- Trägerelemente (24, 25) für jedes Objekt, die in den Beförderungsmitteln integriert sind.

2. Stationäre Handhabungseinrichtung nach einem der vorhergehenden Ansprüche, **dadurch gekennzeichnet, dass** die Isoliermittel durch eine Querschnittsbegrenzung zwischen sich zugewandten Wänden geformt werden, die jeweils zum Rahmen (51) und zum Transferelement (55) gehören.

3. Stationäre Handhabungseinrichtung nach einem der vorhergehenden Ansprüche, **dadurch gekennzeichnet, dass** sie mindestens einen Kanal (54) umfasst, der in den Zwischenbereich mündet und Pumpmitteln zugeordnet ist, um die dem Zwischenbereich zugewandte Zelle unter Vakuum zu setzen.

4. Stationäre Handhabungseinrichtung nach einem der vorhergehenden Ansprüche, **dadurch gekennzeichnet, dass** das Transferelement (55) in der Queransicht mehrere Zellen (56-59) umfasst, im Besonderen vier Zellen, winkelmäßig regelmäßig verteilt.

5. Stationäre Handhabungseinrichtung nach einem der vorhergehenden Ansprüche, **dadurch gekennzeichnet, dass** das Transferelement (55) in der Vorderansicht mehrere Zellen (C1-C12) umfasst, die in der Längsrichtung regelmäßig verteilt sind.

6. Stationäre Handhabungseinrichtung nach einem der vorhergehenden Ansprüche, **dadurch gekennzeichnet, dass** der Abstand zwischen den sich zugewandten Wänden, die jeweils zum Rahmen und zum Transferelement gehören, weniger als 200 Mikrometer ist, im Besonderen weniger als 100 Mikrometer, insbesondere knapp 50 Mikrometer.

7. Stationäre Handhabungseinrichtung nach einem der vorhergehenden Ansprüche, **dadurch gekennzeichnet, dass** die Beförderungsmittel mindestens zwei parallele Förderbänder (11-14) umfassen und mehrere EVE-Trägerelemente (24, 25) nebeneinander gruppiert sind in einem Stützelement (20), das sich transversal zwischen mindestens zwei benachbarten Bändern erstreckt.

8. Stationäre Handhabungseinrichtung nach einem der vorhergehenden Ansprüche, **dadurch gekennzeichnet, dass** die Mittel zum Entstapeln/Stapeln zwei übereinander angeordnete Entstapelelemente (31, 32) umfassen, wobei jedes Element bewegbar ist zwischen einer Halteposition, in der es sich dem Fallen von Objekten entgegensetzt und einer Freigabeposition, in der es das Fallen von Objekten durch die Schwerkraft ermöglicht.

9. Stationäre Handhabungseinrichtung nach dem vorhergehenden Anspruch, **dadurch gekennzeichnet, dass** jedes Entstapelelement einen länglichen Körper (34) umfasst, der mit, besonders halbkreisförmige, gleichmäßig verteilte, Einbuchtungen vertieft ist.

10. Verfahren zur Implementierung der Einrichtung nach einem der vorhergehenden Ansprüche, wobei:
- ein erster Stapel von Objekten (P1) in einer ersten Zelle (56) angeordnet wird, die der Zone bei atmosphärischem Druck (ZA) zugewandt ist,
- das Transferelement so bewegt wird, dass die erste Zelle dem Zwischenbereich (INT) zugewandt ist,
- die erste Zelle, die dem Zwischenbereich zugewandt ist, unter Vakuum gesetzt wird,
- das Transferelement so bewegt wird, dass die erste Zelle der Zone unter Vakuum (ZV) zugewandt ist,
- der erste Stapel von Objekten auf das Entstapelsystem gelegt wird,
- die Objekte des ersten Stapels entstapelt werden,
- jedes so entstapelte Objekt auf ein entsprechendes Trägerelement gelegt wird.

11. Verfahren nach dem vorhergehenden Anspruch, **dadurch gekennzeichnet, dass** die dem Zwischenbereich zugewandte Zelle unter Vakuum gesetzt wird bei einem Druck unter 5.10⁻² mbar, vorzugsweise bei einem Druck unter 10⁻² mbar.

12. Verfahren nach Anspruch 10 oder 11, **dadurch gekennzeichnet, dass** gleichzeitig mit dem Vakuumieren der ersten Zelle, die dem Zwischenbereich zugewandt ist, ein zweiter Stapel von Objekten in einer zweiten Zelle angeordnet wird, die der Zone bei atmosphärischem Druck zugewandt ist.

13. Verfahren nach Anspruch 12, **dadurch gekennzeichnet, dass** gleichzeitig mit dem Vakuumieren der zweiten Zelle, die dem Zwischenbereich zugewandt ist, ein dritter Stapel von Objekten in einer dritten Zelle angeordnet wird, die der Zone bei atmosphärischem Druck zugewandt ist.

14. Verfahren nach einem der Ansprüche 10 bis 13, **dadurch gekennzeichnet, dass** die dem Zwischenbereich zugewandte Zelle für einen Zeitraum zwischen 10 und 60 Sekunden, vorzugsweise zwischen 20 und 40 Sekunden, unter Vakuum gesetzt wird.

## Claims

1. A surface handling facility, comprising:
- a main vacuum enclosure (1) equipped with handling means, in particular of the plasma type,
- at least one device for transferring objects between a zone at atmospheric pressure (ZA) and a zone under vacuum (ZV), the or each transfer device being provided upstream and/or downstream of the main enclosure and comprising:
- a frame (51)
- a housing (L) formed in this frame,
- a transfer member (55), movably mounted in the housing relative to the frame by being rotatably mounted in the housing (L), this transfer member comprising at least one cell (56-59) for receiving at least one object to be transferred,
- the frame defining a first opening (52) in communication with the zone at atmospheric pressure, a second opening (53) in communication with the zone under vacuum, as well as an intermediate region (INT) placed either under vacuum or at atmospheric pressure,
- insulation means between the intermediate region and each opening,
- driving means (60), suitable for driving each cell successively opposite to the first opening, the intermediate region, then the second opening, **characterised in that** the facility further comprises:
- means (30) for unstacking/stacking objects, placed opposite to the second opening of each transfer device, and **in that** the main enclosure of the facility comprises:
- means (11-14) for conveying the objects,
- elements (24, 25) for supporting each object, which are secured to the conveying means.

2. The surface handling facility according to any one of the preceding claims, **characterised in that** the insulation means are formed by a section restriction between opposite walls belonging respectively to the frame (51) and to the transfer member (55).

3. The surface handling facility according to any one of the preceding claims, **characterised in that** it comprises at least one channel (54) opening onto the intermediate region and associated with pumping means, for placing the cell opposite to said intermediate region under vacuum.

4. The surface handling facility according to any one of the preceding claims, **characterised in that**, in transverse view, the transfer member (55) comprises several cells (56-59), in particular four cells, evenly angularly distributed.

5. The surface handling facility according to any one of the preceding claims, **characterised in that**, in front view, the transfer member (55) comprises several cells (C1-C12), evenly longitudinally distributed.

6. The surface handling facility according to any one of the preceding claims, **characterised in that** the distance between the opposite walls, belonging respectively to the frame and to the transfer member, is less than 200 micrometres, in particular less than 100 micrometres, more particularly close to 50 micrometres.

7. The surface handling facility according to any one of the preceding claims, **characterised in that** the conveying means comprise at least two parallel conveying belts (11-14), and several support elements (24, 25) are grouped next to each other within a support member (20), extending transversely between at least two neighbouring bands.

8. The surface handling facility according to any one of the preceding claims, **characterised in that** the unstacking/stacking means comprise two unstacking members (31, 32) placed one above the other, each member being movable between a retaining position, in which it opposes the fall of the objects, and a release position, in which they allow the fall of the objects by gravity.

9. The surface handling facility according to the preceding claim, **characterised in that** each unstacking member comprises an elongate body (34) hollowed out with notches, in particular semi-circular, which are evenly distributed.

10. A method for implementing the facility according to any one of the preceding claims, wherein:
- a first stack of objects (P1) is placed in a first cell (56) opposite to the zone at atmospheric pressure (ZA),
- the transfer member is displaced such that the first cell is opposite to the intermediate region (INT),
- the first cell opposite to the intermediate region is placed under vacuum,
- the transfer member is displaced such that the first cell is opposite to the zone under vacuum (ZV),
- the first stack of objects is deposited on the unstacking system,
- the objects of the first stack are unstacked,
- each object thus unstacked is deposited on a respective support element.

11. The method according to the preceding claim, **characterised in that** the cell opposite to the intermediate region is placed under vacuum, at a pressure below 5.10⁻² mbar, preferably at a pressure below 10⁻² mbar.

12. The method according to claim 10 or 11, **characterised in that**, at the same time that the first cell opposite to the intermediate region is placed under vacuum, a second stack of objects is placed in a second cell opposite to the zone at atmospheric pressure.

13. The method according to claim 12, **characterised in that**, at the same time that the second cell opposite to the intermediate region is placed under vacuum, a third stack of objects is placed in a third cell opposite to the zone at atmospheric pressure.

14. The method according to one of claims 10 to 13, **characterised in that** the cell opposite to the intermediate region is placed under vacuum, for a duration comprised between 10 and 60 seconds, preferably between 20 and 40 seconds.
